# FASCICULE DE BREVET EUROPEEN

(11) **EP 2 121 950 B1**
(45) Date de publication et mention de la délivrance du brevet: **06.04.2011**
(21) Numéro de dépôt: 08761962.3
(22) Date de dépôt: 22.01.2008
(51) Int. Cl.: C12P 7/62

(54) **Procédé d'hydrolyse enzymatique chimiosélectif d'un composé diester pour la préparation d'un composé monoester monoacide**
Verfahren zur chemoselektiven enzymatischen Hydrolyse einer Diester-Verbindung zur Herstellung einer Monoester-Monosäure-Verbindung
Method for the chemo-selective enzymatic hydrolysis of a diester compound for preparing a monoester monoacid compound

(30) Priorité: 22.01.2007 FR 0752792
(43) Date de publication de la demande: 25.11.2009
(73) Titulaire: Zach System, 49240 Avrille (FR); Novacta Biosystems Limited, Hatfield Hertfordshire AL10 9AB (GB)
(72) Inventeur: BURGOS, Alain, F-49130 Les Ponts de Ce (FR); CAILLE, Jean-Claude, F-49000 Angers (FR); LORRAINE GRADLEY, Michelle, Canterbury Kent CT1 3LU (GB)
(74) Mandataire: Nevant, Marc
(86) Numéro de dépôt international: PCT/FR2008/050095
(87) Numéro de publication internationale: WO 2008/110706

(56) Documents cités:
- US-A1- 2004 249 188
- GRELL W ET AL: "REPAGLINIDE AND RELATED HYPOGLYCEMIC BENZOIC ACID DERIVATIVES" JOURNAL OF MEDICINAL CHEMISTRY, AMERICAN CHEMICAL SOCIETY. WASHINGTON, US, vol. 41, 1998, pages 5219-5246, XP000872800 ISSN: 0022-2623 cité dans la demande
- DATABASE EPODOC EUROPEAN PATENT OFFICE, THE HAGUE, NL; YAMAUCHI YOSHIHITO: "1-MONOESTER OF 2-METHYLENEGLUTARIC ACID AND ITS PRODUCTION" XP002438280 -& JP 06 228053 A (IWATA KAGAKU KOGYO) 16 août 1994 (1994-08-16)
- DATABASE WPI Week 199242 Thomson Scientific, London, GB; AN 1992-346220 XP002438282 UEDA WATARU ET AL.: "PRODUCTION OF BENZENEDICARBOXLIC MONOESTER OR DERIVATIVE THEREOF" -& JP 04 252189 A (MERCIAN CORP) 8 septembre 1992 (1992-09-08)

## Description

### Introduction

La présente invention concerne un procédé de synthèse d'un dérivé de l'acide 4-carboxymethyl-2-alkyloxyl benzoïque, alkyl ester à partir du dérivé diester.

Plus spécifiquement, il s'agit d'un procédé de préparation de l'acide 4-carboxymethyl-2-ethoxy benzoïque, éthyle ester, 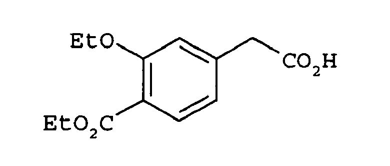 un intermédiaire de synthèse clé pour la production industrielle du principe actif pharmaceutique, le Repaglinide, utilisé dans le traitement du diabète.

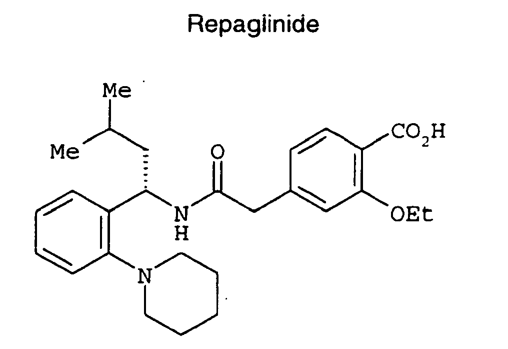

### Art antérieur

On connaît de l'art antérieur, l'article de l'auteur P. Müller et coll. publié dans la revue J. Med. Chem., 1998, 41(26), p 5219, décrivant un procédé de synthèse de l'acide 4-carboxymethyl-2-ethoxy benzoïque, éthyle ester caractérisé par une réaction d'hydrolyse sélective du dérivé diester correspondant.

Cette réaction d'hydrolyse sélective est effectuée dans de l'éthanol à une température de 25 °C et en présence d'une base forte, comme par exemple une solution aqueuse de soude.

Les limites de ce procédé pour une mise en oeuvre industrielle, restent la formation de sous-produits en quantité non négligeable, notamment le composé diacide qui est difficile à éliminer, la nécessité de plusieurs traitements d'extractions et de lavages ou plusieurs recristallisations pour l'isolement du produit et enfin le faible rendement massique d'obtention du produit attendu.

Par ailleurs, l'art antérieur ne décrit pas de procédé de préparation d'un composé acide phenylacétique comprenant une fonction ester sur le noyau aromatique, mettant en oeuvre une réaction d'hydrolyse enzymatique chimiosélective d'un composé diester correspondant.

En particulier, il n'est pas décrit dans l'art antérieur un procédé d'hydrolyse enzymatique chimiosélective d'une fonction ester d'un acide phenylacetique par rapport à une fonction ester d'un acide benzoïque sur un composé comprenant ces deux fonctions.

### Buts de l'invention

La présente invention a pour but principal de résoudre le nouveau problème technique consistant en la fourniture d'un nouveau procédé de préparation de l'acide 4-carboxymethyl-2-ethoxy benzoïque, éthyle ester en évitant la formation de sous-produits et en augmentant le rendement massique.

La présente invention a encore pour but principal de résoudre le nouveau problème technique consistant en la fourniture d'un tel nouveau procédé de manière à minimiser le coût de production.

La présente invention a encore pour but principal de résoudre les nouveaux problèmes énoncés ci-dessus selon un nouveau procédé qui permet de préparer le dit intermédiaire de synthèse en des quantités et une qualité industriellement compatibles avec une production pharmaceutique.

### Résumé de l'invention

La demanderesse a présentement mis au point un nouveau procédé de synthèse du composé de formule (I), tel que défini ci-dessous.

Plus particulièrement, ce procédé est réalisé selon le schéma A ci-dessous, 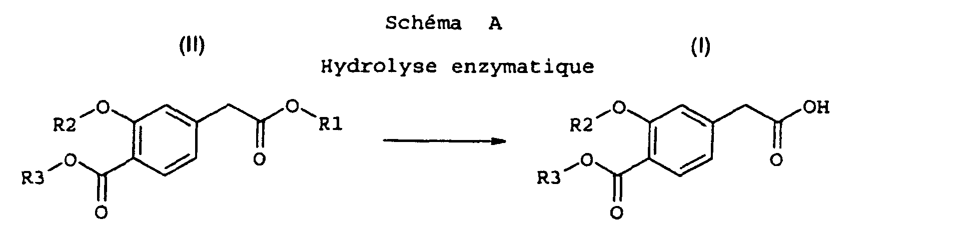 dans lequel R1, R2, R3, identiques ou différents, représentent, individuellement et indépendamment, un groupement alkyle.

Selon un mode de réalisation particulier, chaque groupement R1, R2, R3 est simultanément le même, avantageusement un éthyle.

Dans le cadre de l'invention, le composé de formule (I) est obtenu en mettant en oeuvre une réaction d'hydrolyse enzymatique chimiosélective d'une des deux fonctions esters.

Ce procédé selon l'invention est caractérisé par une réaction d'hydrolyse enzymatique comprenant la mise en contact du composé de formule(II) avec une t.pase réalisant une hydrolyse chimiosélective d'une seule des deux fonctions ester du composé de formule(II) pour obtenir le composé de formule(I). Dans le cadre de l'invention, pour la préparation du Répaglinide, on réalise l'hydrolyse chimiosélective de la fonction ester en position 4 de l'intermédiaire.

Selon un autre mode de réalisation avantageux de ce procédé, la réaction d'hydrolyse enzymatique est effectuée dans une solution à pH contrôlé en présence ou en l'absence d'un co-solvant organique. La valeur du pH de la solution est contrôlée pendant la réaction de telle façon que l'activité de l'enzyme ne soit pas affectée par cette valeur, en particulier cette valeur est maintenue pendant la réaction entre 6 et 8.

Selon encore un autre mode de réalisation avantageux de ce procédé, pour la préparation de l'intermédiaire de synthèse du répaglinide, la réaction d'hydrolyse enzymatique est réalisée à un pH contrôlé par l'emploi d'une solution tampon telle qu'une solution aqueuse à base de phosphate, de carbonate ou de sulfate d'une valeur de pH comprise entre 6 et 8. Plus généralement, la valeur du pH de la solution tampon est telle qu'elle n'affecte pas l'activité de l'enzyme utilisée.

En particulier, la solution tampon est une solution aqueuse ayant une valeur de pH égale à environ 7,2.

Selon un mode de réalisation particulier de l'invention, cette solution tampon aqueuse peut être obtenue par l'emploi d'une solution aqueuse à base de phosphate, de carbonate ou de sulfate, réglée à une valeur de pH initial comprise entre 6 et 8. Une telle solution aqueuse tampon est disponible dans le commerce.

Selon une variante de réalisation, on utilise une solution tampon aqueuse ayant un pH initial d'environ 7,2 en particulier à base de phosphate de potassium.

Selon un autre mode de réalisation particulier de l'invention, le procédé est caractérisé en ce qu'on effectue un contrôle du maintien de la valeur du pH entre 6 et 8 au cours du procédé par l'ajout d'une solution aqueuse d'une base forte telle qu'un hydroxyde ou un alcoolate de métal alcalin.

La normalité de la solution de la base forte est telle qu'elle permet une régulation satisfaisante de la valeur du pH permettant le maintien de l'activité de l'enzyme.

Selon un mode de réalisation avantageux, afin d'assurer le maintien de l'activité de l'enzyme et d'éviter une hydrolyse significative de la deuxième fonction ester, en particulier en position 1 dans le cadre de la fabrication du Répaglinide, le contrôle de la valeur du pH du milieu réactionnel est effectué par l'ajout d'une base forte telle qu'un hydroxyde ou un alcoolate de métal alcalin.

Préférentiellement, le contrôle du pH est effectué par l'ajout d'une solution de soude, plus particulièrement une solution aqueuse de soude. La normalité de la solution basique est telle qu'elle permet une régulation satisfaisante de la valeur du pH permettant le maintien de l'activité de l'enzyme.

Selon un mode de réalisation avantageux de ce procédé selon l'invention, celui-ci est caractérisé en ce que la réaction d'hydrolyse enzymatique est effectuée à une température comprise entre 10°C et 70°C, préférentiellement à une température de environ 45°C.

Selon encore un mode de réalisation avantageux de l'invention, la concentration initiale du produit de départ est comprise entre 1 kg/l et 50 g/l, préférentiellement entre 500 g/l et 50 g/l, très préférentiellement la concentration initiale est d'environ 400 g/l.

Plus généralement la concentration initiale du produit de départ est avantageusement à la valeur sensiblement maximale permettant à la fois la solubilité du produit dans le solvant sélectionné et une non détérioration de l'activité de l'enzyme.

L'enzyme utilisée pour réaliser cette hydrolyse enzymatique sélective, est une lipase.

La lipase utitisée peut être sous une forme solide, en solution, en suspension ou immobilisée sur un support inerte.

L'enzyme peut être directement utilisée soit sous la forme commercialisée soit après une purification.

A titre d'exemple mais non limitatif, on peut utiliser dans le cadre de l'invention une enzyme choisie parmi une lipase, une lipase obtenue à partir d'un Aspergillus (Aspergilus niger, Aspergillus melleus , Aspergillus usuamii), d'un Rhizopus (Rhizopus oryzae, Rhizopus niveus, Rhizopus sp, Rhizopus javenicus, Rhizopus delegar, Rhizopus delemar), d'un Penicillium (Penicillium roquefortii, Penicillium cambertii, Penicillium cyclopium), d'un Mucor (Mucor miehei lyophilisé ou supporté (chirazyme L9), Mucor javanicus, Mucor javenicus), d'un Candida (Candida antartica, Candida antartica type A lyophilisé ou supporté (chirazyme L5), Candida antartica type B lyophilisé ou supporté (chirazyme L2), Candida cylindraceae, Candida lypolytica, Candida rugosa), d'un Pseudomonas (Pseudomonas fluorescens, Pseudomonas cepacia, Pseudomonas aeruginosa, Pseudomonas stutzeri, Pseudomonas species), d'un Alkaligenes (Alcaligenes sp.), d'un Humicola (Humicola lanuginosa, Humicola sp. Lipase), d'un geotrichum (Geotrichum candidum), d'un pancreas (Hog páncreas, porcine páncreas), d'un Achromobacter (Achromobacter sp.), d'un Thermomyces (Thermomyces lanuginose), d'un Burkholderia (Burkholderia cepacia).

Parmi les autres lipases, on peut également citer à titre indicatif mais non limitatif une Lipase F14, une Lipase F10, une Lipase F12, une Lipase A1, une Lipase F7, une Lipase F13, une Lipase F8, une Lipase F2, une Lipase F3, une Amano 6, une Lipase AP15, une Lipase F, une Amano 50, une Lipase G, une Lipase F4, une Lipase F6, une Lipase B1, une Lipase PS-D1, une Lipase PS-C2, une Lipase 1, une Lipase 1, une Lipase 2, une une Euroform Lipase 4, une Lipase 4, une Lipase 6, une Lipase 7, une Lipase 10, une Lipase 11, une Lipase 12, une Lipase 13, une Lipase 14, une Lipase 15, une Lipase 18, une Lipase 20, une Lipase PGE, une Ap-6, une Lipolase.

Ces enzymes sont disponibles commercialement auprès des sociétés telles que Sygma, Amano, Europa, Biocatalysts, Boehringer, Novo-nordisk, Biotal, Enzymatix, Fluka, Genecore, Novorymes etc...

A titre d'exemple mais non limitatif, on peut encore utiliser selon encore une autre variante de réalisation une enzyme commerciale et testée dans le procédé de l'invention, le Candida cylindracea immobilisé (Sigma), le Candida antartica B immobilisé ou le Novozyme 435 (Novozymes), le Mucor miehei immobilisé (Fluka), le Pseudomonas cepacia immobilisé ou le PSD1 (Amano), le Pseudomonas menodicina ou le Lumafast (Genecore), le Humicola languinosa ou le Lipolase, l'Aspergillus niger ou l'AP6 (Amano), le Penicillium camenbertii ou la Lipse G (Amano), le Wheat germ (Fluka), le Rhizopus niveus.

Préférentiellement, l'enzyme choisie est une lipase telle qu'en particulier une enzyme obtenue à partir du Candida antartica type B, par exemple le Novozyme 435 commercialement disponible auprès de la société Novozymes.

Selon un autre mode de réalisation de l'invention, le rapport quantité d'enzyme utilisée et quantité de substrat/produit en valeur poids/poids (le rapport E/S), est compris entre 1/10 000 et 20/100, préférentiellement entre 1/1000 et 5/100.

Selon encore un autre mode de réalisation de l'invention, la concentration d'enzyme peut être comprise entre 1 g/l et de 10 g/l.

Selon une variante de réalisation particulière, la durée d'incubation de l'enzyme est comprise entre 10 minutes et 4 jours, préférentiellement elle est comprise entre 0,5 et 24 h.

Selon encore une autre variante de réalisation particulière, le co-solvant organique éventuellement utilisé, seul ou en mélange, peut être à titre d'exemple mais non limitatif un sulfoxyde comme le dimethylsulfoxyde (DMSO), un nitrile comme l'acétonitrile, un alcool comme l'éthanol, le tertio-butanol, l'isopropanol (IPA), un amide comme le dimethylformamide (DMF), un éther comme l'éther éthylique, un hydrocarbure comme l'hexane, un aromatique comme le toluène.

La concentration du co-solvant peut être comprise entre 0,1% et 30%.

Une fois éliminée lors du traitement de la réaction, l'enzyme peut à nouveau être re-utilisée dans le procédé de l'invention.

Plus généralement le nombre de cycle(s) d'utilisation de l'enzyme peut être entre 1 et 5, préférentiellement entre 1 et 3.

### Définitions

Les définitions ci-dessous sont applicables à la description et aux revendications de l'invention.

Pour faciliter la compréhension, la nomenclature des groupements, des réactifs, des solvants ou des produits est la nomenclature internationale ou la nomenclature couramment utilisée par l'homme du métier.

Le terme alkyle signifie une chaîne alkyle linéaire ou ramifiée, constituée de 1 à 10 atomes de carbones.

Le terme alkoxyde signifie un groupement alkyloxy linéaire ou ramifié, constitué de 1 à 10 atomes de carbones.

Le terme métal alcalin signifie un atome de sodium ou de potassium.

Dans les exemples, les pourcentages sont donnés en poids, la température est en degrés Celsius, la pression est la pression atmosphérique, sauf indication contraire.

Pour faciliter la compréhension, la nomenclature des produits utilisée est la nomenclature internationale et la nomenclature des réactifs ou des solvants est celle couramment utilisée par l'homme du métier.

### Exemple 1 de l'invention: Préparation de_l'acide 4-carboxymethyl-2-ethoxy benzoïque, éthyle ester

Composé de formule (I) dans laquelle R est un groupement éthyle.
Enzyme lipase : Novozym 435
Solution tampon : Solution aqueuse de phosphate de potassium à 0.2 M et de pH 7,2.

La quantité d'enzyme utilisée est de 10 mg.

La réaction est conduite dans une fiole en verre de 4 ml fermée hermétiquement sur une taille de 1 ml.

Le volume réactionnel est fixé à 1 ml en adaptant le volume de solution tampon par rapport à la quantité de matière première utilisée.

La réaction est effectuée à 45°C.

### Exemple 1 a) : 50 mg de diester (concentration 50 g/l).

Après 1 h 50 de réaction, on obtient un taux de conversion de 100%.

### Exemple 1 b) : 100 mg de diester (concentration 100 g/l).

Après 2 h 50 de réaction, on obtient un taux de conversion de 100%.

### Exemple 1 c) : 200 mg de diester (concentration 200 g/l).

Après 3 h 50 de réaction, on obtient un taux de conversion de 80%.

Exemple 2 de l'invention : Préparation de_l'acide 4-carboxymethyl-2-ethoxy benzoïque, éthyle ester :
Composé de formule (I) dans laquelle R est un groupement éthyle.
Enzyme : Novozymes 435
Solution tampon : Solution aqueuse de phosphate de potassium à 0,1 M et de pH 7,2.
Contrôle du pH : Solution aqueuse de NaOH à 2,5 M.

La réaction est conduite dans une fiole en verre de 100 ml fermée hermétiquement. On additionne 3,86 g (13,8 mmol) de diester dans un volume de tampon phosphate de 10 ml et 0,2 g d'enzyme.

On chauffe le milieu à une température de 45°C.

On maintien la valeur du pH à 7,2 par des ajouts réguliers d'une solution de soude.

Après 1 heure 35 mn de réaction, le taux de conversion est de 100%.

L'enzyme est éliminée du milieu par filtration. Le gâteau est lavé à l'eau chaude. Les filtrats sont rassemblés et acidifiés avec une solution d'acide chlorhydrique (HCl) à 20 % jusqu'à l'obtention d'une valeur de pH de 3,5.

Le milieu est mis sous agitation puis refroidi par un bain de glace pendant 1 h. Le précipité formé est filtré puis séché à une température de 45°C jusqu'à l'obtention d'un poids constant.

Le produit obtenu est un solide blanc.
Rendement : Quantitatif
Pureté : 99,3 %

## Revendications

1. Procédé de synthèse du composé de formule (I) selon le schéma A ci-dessous, 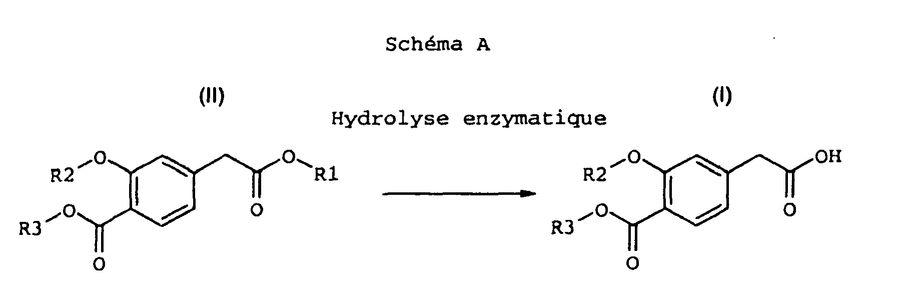 dans lequel R1, R2, R3, identiques ou différents, représentent, individuellement et indépendamment, un groupement alkyle, **caractérisé par** une réaction d'hydrolyse enzymatique comprenant la mise en contact du composé de formule (II) avec une lipase pour obtenir le composé de formule (I).

2. Procédé selon la revendication 1, **caractérisé en ce que** la réaction d'hydrolyse est effectuée dans une solution à pH contrôlé, en particulier par l'emploi d'une solution tampon, en présence ou en l'absence d'un co-solvant organique.

3. Procédé selon la revendication 1 ou 2, **caractérisé en ce que** la lipase utilisée est présente soit sous une forme solide, soit en solution, soit en suspension soit encore sous une forme immobilisée sur un support inerte.

4. Procédé selon l'une des revendications 1 à 3, **caractérisé en ce que** la lipase sélectionnée peut être directement utilisée soit sous la forme commercialisée soit après une purification.

5. Procédé selon l'une des revendications précédentes, **caractérisé en ce que** l'on contrôle pendant la réaction la valeur du pH de telle façon que l'activité de la lipase ne soit pas affectée par cette valeur de pH, en particulier cette valeur de pH est maintenue pendant la réaction entre 6 et 8.

6. Procédé selon l'une des revendications précédentes, **caractérisé en ce que** le nombre de cycle d'utilisation de la lipase choisie est compris entre 1 et 5.

7. Procédé selon l'une des revendications précédentes, **caractérisé en ce que** le rapport quantité de lipase utilisée et quantité de substrat en valeur poids/poids (le rapport E/S), est compris entre 1/10 000 et 20/100, préférentiellement 1/1000 et 5/100.

8. Procédé selon l'une des revendications précédentes, **caractérisé en ce que** la concentration initiale du produit de départ est comprise entre 1 Kg/l et 50 g/l.

9. Procédé selon l'une des revendications précédentes, **caractérisé en ce que** l'hydrolyse enzymatique est conduite à une température comprise entre 10°C et 70°C.

10. Procédé selon l'une des revendications précédentes, **caractérisé en ce que** l'on effectue le contrôle du pH du milieu réactionnel par un ajout de base forte notamment un hydroxyde ou un alcoolate de métal alcalin.

11. Procédé selon l'une des revendications précédentes, **caractérisé par** l'absence d'un co-solvant organique.

12. Procédé selon l'une des revendications précédentes, **caractérisé en ce que** chaque groupement R1, R2 et R3 est simultanément un éthyle.

13. Procédé selon l'une des revendications 1 à 12, **caractérisé en ce que** la lipase utilisée est obtenue à partir du Candida antarctica type B.

14. Procédé selon l'une des revendications 2 à 13, **caractérisé en ce que** la solution tampon est une solution aqueuse à base de phosphate, de carbonate ou de sulfate d'une valeur de pH comprise entre 6 et 8, notamment une solution aqueuse à base de phosphate de potassium.

15. Procédé selon la revendication 14, **caractérisé en ce que** la valeur initiale du pH de la solution tampon est d'environ 7,2.

16. Procédé selon l'une des revendications 2 à 13, **caractérisé en ce que** le contrôle du pH est effectué par l'ajout d'une solution aqueuse d'une base forte, en particulier de soude.

17. Procédé selon l'une des revendications 1 à 16, **caractérisé en ce que** la température de la réaction est préférentiellement d'environ 45°C.

18. Procédé selon l'une des revendications 1 à 17, **caractérisé en ce que** la concentration initiale du produit de départ de formule (II) est d'environ 400 g/l.

19. Procédé selon l'une des revendications 1 à 18, **caractérisé en ce que** la quantité de lipase est comprise entre 1 g/l et 10 g/l.

20. Procédé selon l'une des revendications 1 à 19, **caractérisé en ce que** le composé de formule (I) est utilisé comme intermédiaire de synthèse pour la préparation du Répaglinide.

## Claims

1. A process for synthesizing the compound of formula (I) according to Scheme A below: 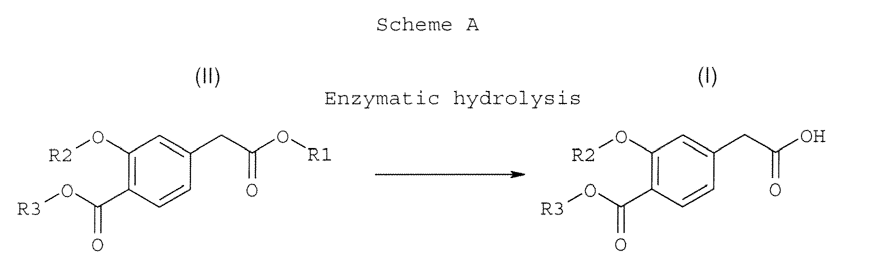 in which R1, R2 and R3, which may be identical or different, represent, individually and independently, an alkyl group, **characterized by** an enzymatic hydrolysis reaction that involves placing the compound of formula (II) in contact with a lipase to obtain the compound of formula (I).

2. The process according to Claim 1, **characterized in that** the hydrolysis reaction is performed in a solution at controlled pH, in particular by using a buffer solution, in the presence or absence of an organic co-solvent.

3. The process according to Claim 1 or Claim 2, **characterized in that** the lipase used is present either in a solid form, or in solution, or in suspension, or in a form immobilized on an inert support.

4. The process according to one of Claims 1 to 3, **characterized in that** the selected lipase may be used directly either in the commercial form or after purification.

5. The process according to one of the preceding claims, **characterized in that** the pH value is controlled during the reaction so that the activity of the lipase is not affected by this pH value, this pH value in particular being maintained between 6 and 8 during the reaction.

6. The process according to one of the preceding claims, **characterized in that** the number of cycles of use of the chosen lipase is between 1 and 5.

7. The process according to one of the preceding claims, **characterized in that** the ratio of the amount of lipase used to the amount of substrate as a weight/weight value (the E/S ratio) is between 1/10 000 and 20/100 and preferentially between 1/1000 and 5/100.

8. The process according to one of the preceding claims, **characterized in that** the initial concentration of the starting material is between 1 kg/l and 50 g/l.

9. The process according to one of the preceding claims, **characterized in that** the enzymatic hydrolysis is performed at a temperature of between 10°C and 70°C.

10. The process according to one of the preceding claims, **characterized in that** control of the pH of the reaction medium is performed by adding a strong base, especially an alkali metal hydroxide or alkoxide.

11. The process according to one of the preceding claims, **characterized by** the absence of an organic co-solvent.

12. The process according to one of the preceding claims, **characterized in that** each group R1, R2 and R3 is simultaneously an ethyl.

13. The process according to one of Claims 1 to 12, **characterized in that** the lipase used is obtained from Candida antarctica type B.

14. The process according to one of Claims 2 to 13, **characterized in that** the buffer solution is an aqueous solution based on phosphate, carbonate or sulphate with a pH value of between 6 and 8, especially an aqueous solution based on potassium phosphate.

15. The process according to Claim 14, **characterized in that** the initial pH value of the buffer solution is about 7.2.

16. The process according to one of Claims 2 to 13, **characterized in that** the pH is controlled by adding an aqueous solution of a strong base, in particular sodium hydroxide.

17. The process according to one of Claims 1 to 16, **characterized in that** the reaction temperature is preferentially about 45°C.

18. The process according to one of Claims 1 to 17, **characterized in that** the initial concentration of the starting material of formula (II) is about 400 g/l.

19. The process according to one of Claims 1 to 18, **characterized in that** the amount of lipase is between 1 g/l and 10 g/l.

20. The process according to one of Claims 1 to 19, **characterized in that** the compound of formula (I) is used as a synthesis intermediate for the preparation of repaglinide.

## Patentansprüche

1. Verfahren zur Synthese der Verbindung der Formel (I) entsprechend nachstehendem Schema A, 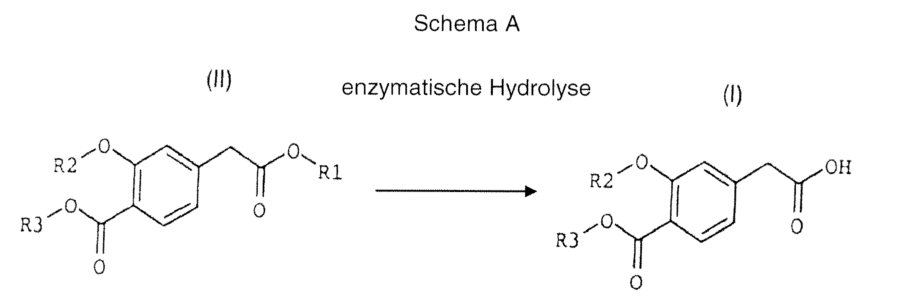 worin R1, R2, R3, die identisch oder unterschiedlich sind, einzeln und unabhängig eine Alkylgruppe darstellen, **gekennzeichnet durch** eine enzymatische Hydrolysereaktion, die das Inkontaktbringen der Verbindung der Formel (II) mit einer Lipase umfaßt, um die Verbindung der Formel (I) zu erhalten.

2. Verfahren nach Anspruch 1, **dadurch gekennzeichnet, daß** die Hydrolysereaktion in einer Lösung mit kontrolliertem pH-Wert, insbesondere unter Verwendung einer Pufferlösung, in Anwesenheit oder in Abwesenheit eines organischen Co-Lösungsmittels durchgeführt wird.

3. Verfahren nach Anspruch 1 oder 2, **dadurch gekennzeichnet, daß** die verwendete Lipase entweder in einer festen Form oder in Lösung oder in Suspension oder aber in einer immobilisierten Form auf einem inerten Träger vorliegt.

4. Verfahren nach einem der Ansprüche 1 bis 3, **dadurch gekennzeichnet, daß** die ausgewählte Lipase direkt verwendet werden kann, entweder in handelsüblicher Form oder nach einer Reinigung.

5. Verfahren nach einem der vorhergehenden Ansprüche, **dadurch gekennzeichnet, daß** während der Reaktion der pH-Wert derart kontrolliert wird, daß die Aktivität der Lipase nicht durch diesen pH-Wert beeinträchtigt wird, insbesondere wird dieser pH-Wert während der Reaktion zwischen 6 und 8 gehalten.

6. Verfahren nach einem der vorhergehenden Ansprüche, **dadurch gekennzeichnet, daß** die Anzahl der Verwendungszyklen der gewählten Lipase zwischen 1 und 5 liegt.

7. Verfahren nach einem der vorhergehenden Ansprüche, **dadurch gekennzeichnet, daß** das Verhältnis zwischen verwendeter Lipase-Menge und Substratmenge in Gewicht/Gewicht (E/S-Verhältnis) zwischen 1/10.000 und 20/100, vorzugsweise zwischen 1/1000 und 5/100 beträgt.

8. Verfahren nach einem der vorhergehenden Ansprüche, **dadurch gekennzeichnet, daß** die Anfangskonzentration des Ausgangsproduktes zwischen 1 kg/l und 50 g/l liegt.

9. Verfahren nach einem der vorhergehenden Ansprüche, **dadurch gekennzeichnet, daß** die enzymatische Hydrolyse bei einer Temperatur zwischen 10 °C und 70 °C durchgeführt wird.

10. Verfahren nach einem der vorhergehenden Ansprüche, **dadurch gekennzeichnet, daß** die Kontrolle des pH-Wertes des Reaktionsmediums durch eine Zugabe einer starken Base, insbesondere eines Hydroxidalkoholats oder eines Alkalimetallalkoholats vollzogen wird.

11. Verfahren nach einem der vorhergehenden Ansprüche, **gekennzeichnet durch** das Nichtvorhandensein eines organischen Co-Lösungsmittels.

12. Verfahren nach einem der vorhergehenden Ansprüche, **dadurch gekennzeichnet, daß** jede Gruppe R1, R2 und R3 gleichzeitig ein Ethyl ist.

13. Verfahren nach einem der Ansprüche 1 bis 12, **dadurch gekennzeichnet, daß** die verwendete Lipase aus Candida antarctica Typ B erhalten wird.

14. Verfahren nach einem der Ansprüche 2 bis 13, **dadurch gekennzeichnet, daß** die Pufferlösung eine wäßrige Lösung auf Phosphat-, Carbonat- oder Sulfatbasis mit einem pH-Wert zwischen 6 und 8, insbesondere eine wäßrige Lösung auf Kaliumphosphatbasis ist.

15. Verfahren nach Anspruch 14, **dadurch gekennzeichnet, daß** der Anfangs-pH-Wert der Pufferlösung etwa 7,2 beträgt.

16. Verfahren nach einem der Ansprüche 2 bis 13, **dadurch gekennzeichnet, daß** die Kontrolle des pH-Wertes durch die Zugabe einer wäßrigen Lösung einer starken Base, insbesondere Natriumhydroxid vollzogen wird.

17. Verfahren nach einem der Ansprüche 1 bis 16, **dadurch gekennzeichnet, daß** die Temperatur der Reaktion vorzugsweise etwa 45 °C beträgt.

18. Verfahren nach einem der Ansprüche 1 bis 17, **dadurch gekennzeichnet, daß** die Anfangskonzentration des Ausgangsproduktes der Formel (II) etwa 400 g/l beträgt.

19. Verfahren nach einem der Ansprüche 1 bis 18, **dadurch gekennzeichnet, daß** die Lipasemenge zwischen 1 g/l und 10 g/l liegt.

20. Verfahren nach einem der Ansprüche 1 bis 19, **dadurch gekennzeichnet, daß** die Verbindung der Formel (I) als Synthesezwischenprodukt für die Herstellung von Repaglinid verwendet wird.
